# NOUVEAU FASCICULE DE BREVET EUROPEEN

(11) **EP 1 207 164 B2**
(45) Date de publication et mention de la décision concernant l'opposition: **25.10.2017**
(45) Mention de la délivrance du brevet: 04.03.2009
(21) Numéro de dépôt: 01402935.9
(22) Date de dépôt: 15.11.2001
(51) Int. Cl.: C07H 15/04, A23G 3/00

(54) **Procédé de production d'une poudre contenant des particles cristallines de maltitol**
Verfahren zur Herstellung von kristallinen Maltitolpartikeln
Method for producing a powder containing crystalline maltitol particles

(30) Priorité: 15.11.2000 US 713539
(43) Date de publication de la demande: 22.05.2002
(73) Titulaire: Roquette Frères, 62136 Lestrem (FR)
(72) Inventeur: Beauregard, Guy, Keokuk, Lee, Iowa 52632 (US); Jorgenson, Mike, Quincy, Adams, Illinois (US); Moser, Ben, Keokuk, Lee, Iowa 52632 (US); Parady, Tom, Hamilton, Hancock, Illinois 62341 (US); Ribadeau-Dumas, Guillaume, 59237 Verlinghem (FR)
(74) Mandataire: Cabinet Plasseraud

(56) Documents cités:
- EP-A- 0 202 165
- EP-A- 0 561 585
- EP-A- 0 741 140
- US-A- 4 408 041
- US-A- 5 583 215

## Description

L'invention concerne un procédé de fabrication continue d'une poudre contenant des particules cristallines de maltitol.

Le maltitol est le résultat de l'hydrogénation du maltose.

On sait déjà comment fabriquer du maltitol cristallisé, par exemple en induisant la cristallisation dudit maltitol dans un sirop suffisamment riche en ce produit et suffisamment purifié.

Les diverses méthodes de la technique antérieure de préparation de cristaux anhydres de maltitol ou d'une poudre contenant des particules cristallines de maltitol comportent la méthode de séparation de mélasses, la méthode de séchage par pulvérisation, la méthode de granulation de fluide ou la méthode de désintégration de blocs.

Cependant, toutes les méthodes ci-dessus présentent un désavantage en ce qu'il est difficile de rendre l'appareillage simple et continu à cause de la longue durée nécessaire à la maturation des cristaux.

US 5 304 388 concerne une méthode de fabrication d'un maltitol cristallin poudreux ou granulé.

Plus précisément, la méthode comprend l'addition de germes cristallins de maltitol à une température inférieure au point de fusion desdits germes cristallins de maltitol dans une solution aqueuse de maltitol ayant 1-15% en poids d'humidité.

Cependant, ce procédé nécessite une étape de pétrissage du mélange en présence ou en absence d'additifs choisis parmi le groupe constitué par des matières grasses, une huile et un agent tensioactif, et particulièrement, il rend critique l'étape d'une application continue d'une force de cisaillement à la masse pétrie.

US 5 583 215 concerne un solide de mélange cristallin contenant du maltitol et un procédé de production de celui-ci.

Le procédé nécessite la fourniture d'une solution aqueuse de maltitol à un extrudeur prévu avec des zones de refroidissement et de pétrissage allongées, le refroidissement et le pétrissage de la solution aqueuse de maltitol en présence de germes cristallins pour former un magma de maltitol, et l'extrusion continue dudit magma depuis une buse.

Cependant, le procédé présente le désavantage de nécessiter l'utilisation d'une solution aqueuse de maltitol ayant une concentration dans la plage de 80 à 98% en poids et une teneur en maltitol dans le composant solide dans la plage de 80 à 99% en poids qui doit être fournie à l'extrudeur équipé de quatre zones successives dont les températures sont précisément contrôlées.

EP 937 733 se concentre sur la réalisation d'améliorations de ces méthodes pour la production continue de mélasses de maltitol contenant des cristaux.

Il décrit une méthode de fabrication comprenant la fourniture continue d'une solution aqueuse de maltitol à un extrudeur ayant une mince zone de pétrissage et de refroidissement, sa soumission à un refroidissement et à un pétrissage continus en présence de germes cristallins pour produire un magma de maltitol qui est extrudé continuellement depuis une buse.

Des étapes ultérieures de désintégration grossière, de maturation, de séchage, etc., sont également mentionnées dans le cadre de la production d'un produit final poudreux.

Cependant, dans ce procédé, le refroidissement est utilisé pour effectuer la cristallisation en augmentant le degré de sursaturation de la solution. Il est donc nécessaire de réduire la viscosité en chauffant la solution ayant une forte concentration en maltitol à une température élevée afin que les germes cristallins soient dispersés de façon homogène en une courte durée.

Ce procédé présente le désavantage d'absorber énormément d'énergie car il nécessite la formation d'une « masse cuite », c'est-à-dire, une substance sursaturée contenant des germes cristallins, ayant une concentration élevée en maltitol, jusqu'à 98% poids/poids de maltitol, et une viscosité qui est abaissée par la fourniture de chauffage à température élevée par un appareil de chauffage au moyen d'une chemise utilisant une vapeur d'eau à environ 110°C et à une pression de 142 kPa.

Il est également nécessaire d'utiliser cet appareil de chauffage particulier pour empêcher la détérioration d'une capacité de mélange et de dispersion résultant d'une augmentation de la viscosité due à un abaissement de la température et il est critique de conserver une faible viscosité afin de disperser et de mélanger les germes cristallins en une durée limitée.

D'autre part, le procédé nécessite une autre étape de désintégration pendant la cristallisation qui rend le procédé plus complexe et prend beaucoup de temps.

De son côté, US 4 408 041 divulgue un procédé complexe de formation de cristaux anhydres de maltitol à partir d'une solution d'amidon liquéfiée, soumise à un traitement enzymatique suivi d'une hydrogénation catalytique, le procédé contenant également des étapes de maturation des cristaux de maltitol ainsi obtenus pendant environ 6 mois pour obtenir une masse cuite.

De plus, toutes les méthodes connues dans la technique antérieure présentent le désavantage d'être onéreuses, complexes et de prendre beaucoup de temps.

L'invention a comme but particulier de fournir un procédé de fabrication d'une poudre contenant des particules cristallines de maltitol qui sont moins sensibles aux désavantages susmentionnés et qui permet d'obtenir efficacement une poudre contenant des particules cristallines de maltitol ayant des propriétés désirables.

Selon la présente invention, on fournit un procédé de fabrication de particules cristallines de maltitol qui ne nécessite pas une très forte concentration de maltitol ou aucun effort entrepris pour contrôler ou mesurer précisément la température pendant l'étape de granulation/cristallisation.

De plus, le procédé de l'invention n'implique pas de formation de masse cuite, ni l'application d'une force de cisaillement ou de pétrissage, reposant plutôt simplement sur un revêtement, une agglomération et une induction de la cristallisation concurrents en permettant au mélange aggloméré de maturer à une température inférieure au point de fusion du maltitol, pour former des granulés solides.

Selon la présente invention, on fournit un procédé de fabrication d'une poudre contenant des particules cristallines de maltitol, comprenant le mélange continu d'un sirop de maltitol ayant une teneur en matière sèche d'au moins 70% en poids et une teneur en maltitol d'au moins 85% en poids sur la base de la matière sèche, le mélange étant effectué en dispersant simultanément le sirop de maltitol et des germes contenant du maltitol dans un récipient rotatif ouvert contenant des granulés à base de maltitol, ce par quoi le sirop de maltitol et les germes contenant du maltitol sont mélangés à la surface des granulés à base de maltitol contenus dans le récipient, la récupération des granulés à base de maltitol depuis le récipient et la cristallisation du maltitol contenu dans lesdits granulés, les granulés à base de maltitol dans le récipient étant maintenus en mouvement par la rotation du récipient.

Lors de la réalisation du procédé de l'invention, le sirop de maltitol est introduit de préférence dans le récipient sous une forme subdivisée par exemple sous forme de gouttes ou de globules, de jets ou de faisceaux de jets.

Selon une méthode préférée de réalisation du procédé susmentionné, un sirop de maltitol, ayant une teneur en matière sèche d'au moins 85% en poids, est porté à une température d'au moins 80°C et est mélangé continuellement dans un récipient avec des germes contenant du maltitol, le rapport germes/sirop, les dimensions, l'orientation de l'axe de rotation et la vitesse de rotation du récipient étant choisis de telle façon que le produit récupéré depuis le récipient apparaît sous la forme de granulés d'un diamètre moyen d'environ 500 à 10 000 µm.

Le procédé de l'invention peut être réalisé dans un appareil comprenant un récipient rotatif ouvert, avec un axe de rotation pouvant être incliné horizontalement, un moyen d'apporter à une zone située à l'intérieur du récipient, au-dessus de la masse qui le remplit partiellement, et d'y disperser une partie de sirop de maltitol, de préférence subdivisé en les formes mentionnées ci-dessus et quelques germes contenant du maltitol, et un moyen d'assurer le mélange du sirop de maltitol et des germes contenant du maltitol à la surface de la masse en mouvement remplissant partiellement le récipient.

Les granulés à base de maltitol sont récupérés de préférence par débordement à la sortie du récipient et peuvent être maturés pour augmenter leur cristallinité par le transfert des granulés dans un cylindre rotatif de caractéristiques dimensionnelles telles que la durée du séjour des granulés provenant du récipient est suffisante pour assurer la cristallisation du maltitol. Les granulés peuvent ensuite être transférés dans un séchoir pour diminuer l'humidité résiduelle et ensuite dans un moyen de broyage et de criblage.

Lors de la réalisation de procédé de l'invention, un sirop de maltitol, du type disponible dans le commerce, c'est-à-dire pouvant contenir d'autres polyalcools tels que le sorbitol, et dont la matière sèche est d'au moins 70%, et ayant une teneur en maltitol d'au moins 85% sur la base de la matière sèche, est dispersé à une température d'environ 80°C à l'intérieur d'un récipient rotatif ouvert sous forme d'un réservoir ou d'un tambour ouvert ayant un fond essentiellement plat, dont l'axe de rotation peut être incliné sur un plan horizontal d'un angle de 25 à 45°.

Une buse atomisation d'air a été avantageusement utilisée pour pulvériser le sirop aqueux sur le lit rotatif de matériaux de germes contenant du maltitol, dans le granulateur à échelle pilote.

En ce qui concerne le transfert des germes contenant du maltitol dans le mélange susmentionné et dont les particules constituantes servent de germes contenant du maltitol cristallisé, on préfère une taille de particules d'environ 100 µm.

Le rapport pondéral dans le mélange constitué de germes contenant du maltitol et de sirop de maltitol est d'environ 4/1.

Le mélange est effectué à la surface de la masse en mouvement remplissant partiellement le récipient ; le mouvement en question rappelle une masse de pilules à l'intérieur d'une machine de fabrication de pilules, et on a constaté que des granulés qui sont de plus en plus gros sont formés, les granulés les plus grands ayant tendance à venir à la surface de la masse en mouvement.

Les granulés de maltitol ainsi obtenus sont ensuite maturés pour augmenter leur cristallinité.

Cette étape de maturation peut être réalisée en conservant les granulés en mouvement à une température inférieure au point de fusion des granulés, préférablement à une température de 5 à 90°C, pendant de 1 à 20 heures dans un courant d'air.

Le produit granulé est ensuite séché afin d'obtenir une humidité résiduelle non supérieure à environ 2%.

Les granulés peuvent ensuite être broyés jusqu'à la taille de particules requise et ensuite triés par criblage ; les particules éliminées par criblage peuvent ensuite être avantageusement recyclées vers le récipient susmentionné pour une utilisation comme germes contenant du maltitol.

Les poudres résultantes contenant des particules cristallines de maltitol sont caractérisées par le fait qu'elles présentent une vitesse de solubilisation dans l'eau, selon le test A, inférieure à 3 min.

Afin de mesurer la première caractéristique de la poudre contenant des particules cristallines de maltitol selon le procédé de l'invention, à savoir le temps de solubilisation, on réalisera le test A. Le test A consiste à introduire, dans 500 ml d'eau déminéralisée et dégazée, maintenue à 37°C, et soumise à une agitation à 100 rpm, exactement 5 g d'une section de taille de particules de 75 µm à 150 µm du produit à tester.

Le temps de solubilisation correspond au temps nécessaire, après introduction de la section de taille de particules, pour l'obtention d'une clarté visuelle parfaite du mélange ainsi préparé.

Dans ces conditions, la poudre contenant des particules cristallines de maltitol obtenues selon le procédé conforme à l'invention a généralement une vitesse de solubilisation inférieure à 3 min. Le produit préféré se solubilise en moins de 2,0 min.

Ces temps sont inférieurs à ceux obtenus avec d'autres poudres de maltitol actuellement commercialisées.

Une deuxième propriété très avantageuse est que la poudre contenant des particules cristallines de maltitol obtenues selon le procédé conforme à l'invention présente une valeur de compression, selon le test B, qui est jusqu'à environ 500 N.

Le test B consiste à mesurer la dureté, exprimée en Newtons, d'un échantillon de comprimé produit sur une Presse Carver par une charge de 18 kNewtons appliquée à une matrice annulaire Carver n° 3619 de 13 mm pour obtenir un comprimé contenant approximativement 0,9 g de poudre contenant des particules cristallines de maltitol obtenues selon le procédé conforme à l'invention et 1% de stéarate de magnésium.

La dureté du comprimé est déterminée dans un testeur de comprimés du Dr SCHLEUNIGER PHARMATRON modèle 6D.

Les résultats montrent que la poudre contenant des particules cristallines de maltitol obtenues selon le procédé conforme à l'invention présente une valeur de dureté élevée.

Afin de mesurer la gravité spécifique apparente de la poudre contenant des particules cristallines de maltitol obtenues selon le procédé conforme à l'invention, on réalisera le test C.

Le test C consiste à verser approximativement 250 ml de matériau ayant une section de taille de particules de 300 µm à 850 µm dans une éprouvette graduée de 250 ml, et les valeurs de gravité spécifique apparente sont calculées en divisant le poids du matériau par son volume mesuré.

Les performances de la poudre contenant des particules cristallines de maltitol obtenues selon le procédé conforme à l'invention conforme à l'invention sont évaluées dans des biscuits secs sablés sans sucre, dans des biscuits secs sans sucre au gruau d'avoine emmoulés et dans du chewing-gum.

Les exemples suivants illustrent la préparation de la poudre contenant des particules cristallines de maltitol par l'utilisation du procédé selon l'invention.

### Exemple 1

Une solution ayant une teneur d'environ 89,9% en maltitol sur la base de la matière sèche est placée dans un récipient d'évaporation pour obtenir un sirop de maltitol ayant une teneur en matière sèche d'environ 90%.

Ce sirop de maltitol est placé dans un réservoir de stockage à une température d'environ 115°C, à partir duquel il est continuellement prélevé au moyen d'une pompe qui assure sa dispersion sous forme de globules au moyen d'une buse.

Simultanément à la dispersion dudit sirop de maltitol, on introduit continuellement du matériau de germes supplémentaire dans le granulateur à échelle pilote pour réaliser un rapport pondéral de germes/sirop d'environ 4 parties de germes pour 1 partie de sirop de maltitol.

Les germes sont obtenus par le recyclage continu d'une fraction du matériau solidifié étant produit. Des particules d'un solide de maltitol cristallin quelconque peuvent être utilisées pour fournir des germes pour la granulation initiale.

Aucun effort particulier n'est entrepris pour contrôler la température du granulateur.

Le granulateur tourne à une vitesse d'environ 6,5 tours par minute, son inclinaison étant de 30°, ce qui permet d'obtenir des granulés ayant un diamètre moyen d'environ 500 µm à 10 000 µm.

Après cette étape de granulation, lesdits granulés sont maturés par l'achèvement de la cristallisation dans un dispositif de maturation (tambour rotatif allongé).

Les granulés maturés ainsi obtenus sont soumis à un broyage grossier et séchés dans un lit fluidisé en utilisant de l'air à environ 90°C.

Après le séchage, ils apparaissent sous la forme d'une poudre contenant environ 1,8% d'humidité résiduelle.

A la sortie du transporteur à rouleaux du lit fluidisé, la poudre séchée est conduite à une installation classique de broyage.

La poudre séchée est ensuite criblée et une partie en est recyclée comme germes contenant du maltitol vers le granulateur.

La poudre restante est la poudre contenant des particules cristallines de maltitol obtenues selon le procédé selon l'invention.

La vitesse de solubilisation, telle que mesurée selon le test A, est de 1 minute et 50 secondes, et la valeur de dureté déterminée selon le test B est de 470 N.

La gravité spécifique apparente, telle que mesurée selon le test C, est de 0,602 g/ml.

### Exemple 2

Les performances de la poudre contenant des particules cristallines de maltitol de l'Exemple 1 ont été évaluées dans des biscuits secs sablés sans sucre et dans des biscuits secs sans sucre au gruau d'avoine emmoulés.

MALTISORB® P200 commercialisé par ROQUETTE FRERES et AMALTY® MR-50 de chez TOWA ont été utilisés pour produire des exemples comparatifs. Les ingrédients des biscuits secs sablés sans sucre et des biscuits secs sans sucre au gruau d'avoine sont les suivants.

| **BISCUITS SECS SABLES SANS SUCRE** | |
|---|---|
| **INGREDIENT** | **Pourcentage** |
| Maltitol | 18,00 |
| Matière Grasse (ADM Cookie Bake) | 21,70 |
| Sel | 0,50 |
| Bicarbonate de Soude | 0,16 |
| Arôme Vanille (Massey Bourbon) | 0,30 |
| Oeuf Entier | 0,67 |
| Sorbitol P 110 | 2,00 |
| Levure Chimique | 0,04 |
| Arôme Naturel de Beurre (Bell Flavors OS) | 0,04 |
| Colorant Caramel | 0,06 |
| Eau | 8,00 |
| Farine de Pâtisserie (Con Agra) | 48,53 |
| | 100,00 |

Le mode opératoire est le suivant :
Malaxer le Sorbitol, le Maltitol, la Matière grasse, le Sel, le Bicarbonate de Soude, l'Arôme, l'Oeuf et la Levure Chimique à faible vitesse pendant 2 min.
Ajouter l'arôme de Beurre, le Colorant et l'eau. Pétrir pendant 1 min à faible vitesse.
Ajouter la farine et pétrir pendant 1 min à faible vitesse.
Cuire à 425°F pendant 9 min.

| **BISCUITS SECS AU GRUAU D'AVOINE SANS SUCRE** | |
|---|---|
| **INGREDIENT** | **Pourcentage** |
| Maltitol | 18,60 |
| Matière Grasse « Cookie Bake » | 16,00 |
| Oeufs | 0,50 |
| Arôme Vanille (Massey Bourbon) | 0,16 |
| Bicarbonate de Soude | 0,36 |
| Cannelle | 0,21 |
| Sel | 0,20 |
| Arôme de Sucre Brun | 0,17 |
| Neosorb Sorbitol P 110 | 6,80 |
| Avoine « Quick » [Avoine partiellement broyée] | 20,40 |
| Farine de Pâtisserie (Con Agra) | 29,09 |
| Eau | 7,51 |
| | 100,00 |

Le mode opératoire est le suivant :
Malaxer le maltitol, le sorbitol, la matière grasse, le sel, le bicarbonate de soude et l'oeuf à faible vitesse pendant 2 min.
Ajouter la moitié du mélange de sucre brun et de l'arôme vanille et de l'eau. Pétrir pendant 1 min à faible vitesse.
Ajouter la farine, l'avoine et le reste du mélange d'eau et pétrir pendant 1 min à faible vitesse.
Préparer des lots de 22 - 23 g par biscuit sec et cuire à 425°F pendant 9 min.

Dans les biscuits secs sablés et au gruau d'avoine, la poudre contenant des particules cristallines de maltitol obtenues selon le procédé de l'invention a présenté une solubilisation plus rapide, qui a permis une réduction du temps de pétrissage depuis un total de 6 min à 4 min.

Dans la recette de sablés, la texture de la pâte issue de ce mode opératoire était comparable à celle d'une pâte faite avec MALTISORB® P200 et a été facilement transformée dans un procédé de moulage pour former des biscuits secs de forme et de taille désirées. Les biscuits secs au gruau d'avoine faits avec la poudre contenant des particules cristallines de maltitol obtenues selon le procédé de l'invention ont également nécessité moins de temps de pétrissage pour atteindre la même consistance typique de biscuits secs emmoulés ou coupés au fil.

Aucune modification des conditions de cuisson pour l'un ou l'autre des types de biscuit sec n'a été faite ou n'a été nécessaire. Les biscuits secs faits avec la poudre contenant des particules cristallines de maltitol obtenues selon le procédé de l'invention sont plus légers que des biscuits secs sans sucre comparables faits avec MALTISORB® P200 et AMALTY® R50.

Lors d'un stockage prolongé, les biscuits secs faits avec la poudre contenant des particules cristallines de maltitol obtenues selon le procédé de l'invention sont restés plus mous que les biscuits secs témoins faits avec MALTISORB® P200 et AMALTY® R50, une caractéristique désirée pour des produits ayant une longue durée de conservation.

### Exemple 3

Des compositions de chewing-gum aromatisé ont été préparées selon les deux formulations de base ci-dessous, dans lesquelles le maltitol utilisé est soit des particules cristallines de maltitol obtenues selon le procédé selon l'invention (composition A), ayant un diamètre moyen de taille de particules de 250 µm, soit du MALTISORB® P35, commercialisé par la Société Demanderesse (composition B), ayant un diamètre moyen de taille de particules de 35 µm (taille de particules recommandée pour la fabrication de chewing-gum).

| | Composition A | Composition B |
|---|---|---|
| | Pourcentage | Pourcentage |
| Base de gomme HORIZON® (CAFOSA GUM S\A) | 25 | 25 |
| MALTISORB® P35 | | 60,8 |
| Particules cristallines de maltitol obtenues selon le procédé de l'invention | 60,8 | |
| LYCASIN® 80/55 concentré (85%) commercialisé par la Société Demanderesse | 12 | 12 |
| Arôme naturel de menthe (Silesia) | 2,2 | 2,2 |
| | 100.0 | 100,0 |

La base de gomme et la moitié soit des particules de maltitol obtenues selon le procédé selon l'invention, soit du MALTISORB® P35, sont introduites dans un mélangeur-malaxeur équipé de deux bras en Z et d'une chemise, maintenu à une température de 50°C par de l'eau circulant dans la chemise.

Après malaxage de la base de gomme pendant environ 3 minutes, environ la moitié du LYCASIN® 80/55 est ajoutée et pétrie intimement avec la base de gomme.

Le mélange est malaxé continuellement pendant 3 minutes, suivi par l'addition de la quantité restante soit des particules cristallines de maltitol selon l'invention, soit du MALTISORB® P35.

Après environ 6 minutes de malaxage supplémentaire, on ajoute la quantité restante du LYCASIN® 80/55.

Enfin, après malaxage pendant 4 minutes, on introduit l'arôme de menthe.

On continue le malaxage pendant encore 1 minute de manière à obtenir une pâte homogène.

Cette pâte est ensuite retirée du pétrin mécanique, laminée et découpée en bâtonnets de 5 millimètres d'épaisseur.

### 1. Mesure de la dureté

On a établi la comparaison, pour les deux compositions de chewing-gum, entre la dureté de bâtonnets de 5 millimètres d'épaisseur, âgés de 8 jours et conservés à une humidité de 50% à 20°C, par pénétrométrie en utilisant une machine de marque INSTRON®.

Les résultats moyens de dureté (valeurs moyennes pour dix bâtonnets) étaient comme suit :
Composition A : 63,7 N
Composition B : 73,6 N
On observe que la composition de chewing-gum A est légèrement plus molle que la composition B.

### 2. Evaluation du goût

Dans un test en aveugle, on a demandé à un panel de 25 personnes de classer les trois compositions de chewing-gum de 0 à 4 par ordre de préférence (note 0 pour le produit considéré comme ayant le goût le moins bon et note 4 pour le produit préféré).

Une troisième composition (composition C) a été faite avec du MALTISORB® P200 (ayant une taille de particules de maltitol du même ordre de grandeur que les particules cristallines de maltitol obtenues selon le procédé selon l'invention) de manière semblable aux compositions A et B.

Ces notes ont ensuite été associées et interprétées, en prenant en compte les commentaires de chaque panéliste.

La composition C a obtenu la note la plus basse.

Ceci est expliqué par la texture « sableuse » des bâtonnets, en comparaison avec ceux fabriqués avec les compositions A et B.

Ce goût sableux pouvait être expliqué par la granulométrie de MALTISORB® P200, utilisé comme ingrédient dans la composition C.

De manière surprenante, les bâtonnets faits avec la composition A, ayant une taille de particules de maltitol de valeur semblable à celle du MALTISORB® P200, sont d'un goût équivalent à celui de bâtonnets faits avec la composition B.

La composition préférée A a un goût non sableux, est très moelleuse en bouche, et a obtenu des notes comprises entre 3,5 et 3,8, qui sont remarquables.

La conclusion est que les particules cristallines de maltitol obtenues selon le procédé selon l'invention sont particulièrement adaptées à la fabrication du chewing-gum.

## Revendications

1. Procédé de fabrication d'une poudre contenant des particules cristallines de maltitol, comprenant le mélange continu d'un sirop de maltitol ayant une teneur en matière sèche d'au moins 70% en poids et une teneur en maltitol d'au moins 85% en poids sur la base de la matière sèche, le mélange étant effectué en dispersant simultanément le sirop de maltitol et des germes contenant du maltitol dans un récipient rotatif ouvert contenant des granulés à base de maltitol, ce par quoi le sirop de maltitol et les germes contenant du maltitol sont mélangés à la surface des granulés à base de maltitol contenus dans le récipient, la récupération des granulés à base de maltitol depuis le récipient et la cristallisation du maltitol contenu dans lesdits granulés, les granulés à base de maltitol dans le récipient étant maintenus en mouvement par la rotation du récipient.

2. Procédé selon la revendication 1, dans lequel le sirop de maltitol est introduit dans le récipient sous forme subdivisée.

3. Procédé selon la revendication 2, dans lequel le sirop de maltitol est introduit sous forme de gouttes.

4. Procédé selon la revendication 2, dans lequel le sirop de maltitol est introduit sous forme de jets.

5. Procédé selon la revendication 1, dans lequel l'axe de rotation du récipient est incliné par rapport à l'horizontale.

6. Procédé selon la revendication 1, dans lequel les granulés à base de maltitol sont récupérés par débordement à la sortie du récipient.

7. Procédé selon la revendication 1, dans lequel des granulés d'un diamètre de 150 à 10 000 µm sont récupérés.

8. Procédé selon la revendication 1, dans lequel un sirop de maltitol, ayant une teneur en matière sèche d'au moins 90% en poids, est porté à une température d'au moins 80°C et est mélangé continuellement avec des germes contenant du maltitol, le rapport germes/sirop, les dimensions, l'orientation de l'axe de rotation et la vitesse de rotation du récipient étant choisis de telle façon que le produit récupéré depuis le récipient apparaît sous la forme de granulés ayant un diamètre de 150 à 10 000 µm.

9. Procédé selon la revendication 8, dans lequel le rapport germes/sirop n'est pas supérieur à environ 4/1.

10. Procédé selon le revendication 1, dans lequel le récipient est sous le forme d'un réservoir ou d'un tambour ouvert ayant un fond essentiellement plat

11. Procédé selon le revendication 10, dans lequel l'axe de rotation du récipient fait un angle de 25 à 45° par rapport à l'horizontale.

12. Procédé selon le revendication 1, dans lequel les granulés de maltitol récupérés sont maturés afin d'augmenter leur cristallinité en maintenant les granulés à une température de 5 à 90°C pendant de 1 à 20 heures tout en maintenant les granulés en mouvement dans un courant d'air.

13. Procédé selon la revendication 12, dans lequel les granulés de maltitol maturés récupérés sont broyés et séchés afin d'obtenir une humidité résiduelle non supérieure à environ 2%.

## Patentansprüche

1. Verfahren zur Herstellung eines Pulvers aufweisend Kristallpartikel aus Maltitol, umfassend die kontinuierliche Mischung von einem Maltitolsirup aufweisend einen Trockenmassegehalt von mindestens 70 Gew.-% und einen Maltitolgehalt von mindestens 85 Ges.-% auf der Grundlage der Trockenmasse, wobei die Mischung durch das gleichzeitige verteilen des Maltitolsirups und Maltitol enthaltender Keime in einen umlaufenden offenen Behälter ausgeführt wird, der Körnchen auf Maltitolbasis enthält, wobei der Maltitolsirup und die Maltitol enthaltenden Keime an der Flache der in dem Behälter enthaltenen Körnchen auf Maltitolbasis gemischt werden, und die Rückgewinnung der Körnchen auf Maltitolbasis in dem Behälter und das Kristallisieren des Maltitols, der in den Körnchen enthalten ist, wobei die Körnchen auf Maltitolbasis in dem Behälter durch die Umlaufbewegung des Behälters in Bewegung gehalten werden.

2. Verfahren nach Anspruch 1, wobei der Maltitolsirup in unterteilter Form in den Behälter eingeführt wird.

3. Verfahren nach Anspruch 2, wobei der Maltitolsirup in Form von Tropfen eingeführt wird.

4. Verfahren nach Anspruch 2, wobei der Maltitolsirup in Form von Strahlen eingeführt wird.

5. Verfahren nach Anspruch 1, wobei die Rotationsachse des Behälters in Bezug auf die Horizontale geneigt ist.

6. Verfahren nach Anspruch 1, wobei die Körnchen auf Maltitolbasis rückgewonnen werden, indem sie an den Ausgang des Behälters überlaufen.

7. Verfahren nach Anspruch 1, wobei die Körnchen, die einen Durchmesser von 150 bis 10.000 µm aufweisen, rückgewonnen werden.

8. Verfahren nach Anspruch 1, wobei ein Maltitolsirup aufweisend einen Trockenmassegehalt von mindestens 90 Ges.-% bei einer Temperatur von mindestens 80°C gehalten und kontinuierlich mit Keimen gemischt wird, die Maltitol enthalten, wobei das Verhältnis von Keimen zu Sirup, die Größen, die Ausrichtung der Rotationsachse und die Umlaufgeschwindigkeit des Behälters so gewählt werden, dass das Produkt, das in dem Behälter rückgewonnen wird, in Form von Körnchen mit einem Durchmesser von 150 bis 10.000 µm auftritt.

9. Verfahren nach Anspruch 8, wobei das Verhältnis von Keimen zu Sirup nicht größer als circa 4:1 ist.

10. Verfahren nach Anspruch 1, wobei der Behälter die Form einer Wanne oder einer offenen Trommel mit einem im Wesentlichen flachen Boden aufweist.

11. Verfahren nach Anspruch 10, wobei die Rotationsachse des Behälters einen Winkel von 25 bis 45° in Bezug auf die Horizontale aufweist.

12. Verfahren nach Anspruch 1, wobei die rückgewonnenen Maltitolkörnchen gereift werden, um ihre Kristallinität zu erhöhen, indem die Körnchen für 1 bis 20 Stunden bei einer Temperatur zwischen 5 und 90°C gehalten werden, wobei die Körnchen in einem Luftstrom in Bewegung gehalten werden.

13. Verfahren nach Anspruch 12, wobei die gereiften und rückgewonnenen Maltitolkörnchen zerkleinert und getrocknet werden, um eine Restfeuchtigkeit von nicht mehr als circa 2% zu erhalten.

## Claims

1. Process for producing a powder containing crystalline maltitol particles, including the continuous mixing of a maltitol syrup having a dry solids content of at least 70% by weight and a maltitol content of at least 85% by weight on the base of the dry solids, in which the mixing is performed by simultaneously dispersing the maltitol syrup and seeds containing maltitol in an open rotary container containing maltitol-based granules, whereby the maltitol syrup and the seeds containing maltitol are mixed at the surface of the maltitol-based granules contained in the container, and the recovery of the maltitol-based granules from the container and the crystallization of the maltitol contained in said granules, in which the maltitol-based granules in the container are kept in motion by the rotation of the container.

2. Process according to claim 1, in which the maltitol syrup is introduced into the container in subdivided form.

3. Process according to claim 2, in which the maltitol syrup is introduced in the form of drops.

4. Process according to claim 2, in which the maltitol syrup is introduced in the form of jets.

5. Process according to claim 1, in which the axis of rotation of the container is tilted with respect to the horizontal.

6. Process according to claim 1, in which the maltitol-based granules are recovered by overflew at the outlet of the container.

7. Process according to claim 1, in which the granules with a diameter of 150 to 10,000 µm are recovered.

8. Process according to claim 1, in which a maltitol syrup, having a solids content of at least 90% by weight, is brought to a temperature of at least 80°C and is continuously mixed with seeds containing maltitol, in which the seed/syrup ratio, the size, the orientation of the axis of rotation and the speed of rotation of the container are chosen so that the produced recovered from the container appears in the form of granules with a diameter of 150 to 10,000 µm.

9. Process according to claim 8, in which the seed/syrup ratio is no greater than around 4/1.

10. Process according to claim 1, in which the container is in the form of a tank or an open drum having an essentially flat base.

11. Process according to claim 10, in which the axis of rotation of the container formes an angle of 25 to 45° with respect to the horizontal.

12. Process according to claim 1, in which the recovered maltitol granules are matured so as to increase their crystallinity by keeping the granules at a temperature of 5 to 90°C for 1 to 20 hours while keeping the granules in motion in an air current.

13. Process according to claim 12, in which the recovered mature maltitol granules are around and dried so as to obtain a residual moisture no greater than around 2%.
